# EUROPEAN PATENT APPLICATION

(11) **EP 1 679 059 A2**
(43) Date of publication of application: **12.07.2006**
(21) Application number: 03759058.5
(22) Date of filing: 28.10.2003
(51) Int. Cl.: A61K 6/00

(54) **UTILIZATION OF VINPOCETINE TO AVOID COMPLICATIONS IN PARTICULAR THOSE ASSOCIATED TO HEARING WHICH OCCUR WITH EPILEPSY, AND TREATMENT THEREOF**

(71) Applicant: UNIVERSIDAD NACIONAL AUTONOMA DE MEXICO, México, D.F. 04510 (MX)
(72) Inventor: SITGES BERRONDO, Maria, México, D.F. 4650 (MX); NEKRASSOV PROTASOVA, Vladimir, México, D.F. 04650 (MX)
(74) Representative: Carvajal y Urquijo, Isabel
(86) International application number: PCT/MX2003/000089
(87) International publication number: WO 2005/039482

(57) **Abstract**

The present invention is related with the use of vinpocetine and the derivates developed from its formula that maintains the same effects for the treatment of epilepsy and its complications. Our results show that vinpocetine prevents all the abnormalities of the ABR waves that accompany the epileptic cortical activity observed for the ictal and post-ictal periods in two experimental models of epilepsy in vivo, that vinpocetine also inhibits the marked hearing loss and the characteristic EEG changes induced by two convulsing agents that differ in their mechanisms of action. These findings also indicate that the capacity of vinpocetine as an antiepileptic drug is not accompanied by adverse secondary effects.

## Description

### TECHNICAL FIELD

The present invention is related with the use of vinpocetine and the derivates developed from its formula that maintain the same effects for the treatment of epilepsy and its complications, particularly those related with the auditory pathway.

### BACKGROUND

One major problem of epilepsy is the deleterious cognitive and behavioural consequences caused by the illness (Prevey et al. 1998 *Epilepsy Res.* 30: 1; Jokeit and Ebner 1999 *J. Neurol. Neurosurg. Psychiatry* 67: 44; Theodore et al. 1999 *Neurology* 52: 132; Meador 2001 *Epilepsy Behav.* 2: 307) as well as by its treatment with the available antiepileptic drugs (Gates 2000 *Epilepsy Behav.* 1: 153; Kwan and Brodie 2001 *Lancet* 357: 216; Brunbech and Sabers 2002 *Drugs* 62: 593; Schmidt 2002 *Epilepsy Res.* 50: 21).

The implication of the auditory brainstem nuclei in the pathophysiology of generalized epilepsy is indicated by the alterations in the latencies and/or amplitudes of the later waves of the auditory brainstem response (ABR) observed in patients with generalized epilepsy (Rodin et al. 1982 *Clin. Electroencephalogr.* 13: 154; Mervaala et al. 1986 *Epilepsia* 27: 542; Phillips et al. 1990 *Clin. Electroencephalogr.* 21: 135; Soliman et al. 1993 *Ear Hear* 14: 235; Kohsaka et al. 1999 *Brain Res.* 837: 277; Kohsaka et al. 2001 *Brain Res.* 903: 53). The acute epilepsy induced by two convulsive agents in non medicated animals is accompanied of abnormalities in the later waves of the ABR and by a marked hearing decline (Nekrassov and Sitges 2003 *Epilepsy Res.* 53: 245).

ABRs are far field-evoked potentials that consist of several waves that occur within 10 ms after the auditory stimulus. Since changes in the later waves of the ABR indicate alterations of specific nuclei of the auditory brainstem (Hughes, J.R., Fino, J.J., 1985 *J*. *Clin. Neurophysiol.* 2: 355), ABRs are commonly used in the clinical diagnosis of retro-cochlear lesions. In addition, the ABR threshold is used in the clinical diagnosis of the hearing sensitivity, because stimuli of progressively higher intensity (in dB) are needed for evoking the ABR while the hearing sensitivity declines. Therefore elevations in the ABR thresholds are an objective determination of hearing deterioration.

Antiepileptic drugs, including carbamazepine, valproate, phenytoin, phenobarbital, clonazepam and vigabatrin, also cause abnormalities on the waves of the ABR as well as hearing deficits (Mervaala et al. 1987 *Electroencephalogr. Clin. Neurophysiol.* 68: 475; Armon et al. 1990 *Neurology* 40: 1896; Hirose et al. 1990 *Electroencephalogr. Clin. Neurophysiol.* 75: 543; Yuksel et al. 1995 *Childs Nerv. Syst.* 11: 474; De la Cruz and Bance 1999 *Arch Otolaryngol Head Neck Surg.* 125: 225; Zgorzalewicz and Galas-Zgorzalewicz 2000 *Clin. Neurophysiol.* 111:2150).

Vinpocetine (ethyl apovincamine-22-oate) discovered during the late 1960s has successfully been used in the treatment of central nervous system disorders of cerebrovascular origin for decades. In animal models of hypoxia and ischemia vinpocetine exerts beneficial effects against neuronal damage (King 1987 *Arch. Int. Pharmacodyn. Ther.* 286:299; Araki et al. 1990 *Res..Exp..Med.* 190:19).

More recently vinpocetine has also been used for ameliorating memory on the basis of previous studies in animals and humans (Subhan and Hindmarch 1985 *Eur. J. Clin. Pharmacol.* 28: 567; Bhatti and Hindmarch 1987 *Int. Clin. Psychopharmacol.* 2: 325; DeNoble 1987 *Pharmacol. Biochem. Behav.* 26: 183).

Vinpocetine is a Na⁺ channel blocker (Erdö et al. 1996 *Europ. J. Pharmacol.* 314:69). In brain isolated nerve endings we have shown that vinpocetine selectively inhibits neurotransmitter release induced by an increase in presynaptic Na⁺ channels permeability (Sitges and Nekrassov, 1999 *Neurochem. Res.* 24:1587; Trejo et al. 2001 *Brain Res.* 909:59). In addition we have shown that in the guinea pig in vivo vinpocetine exerts a long term inhibition of the alterations in the ABR waves, the hearing loss and the mortality induced by amikacin (Nekrassov and Sitges 2000 *Brain Res.* 868: 222) and other aminoglycoside antibiotics (unpublished results).

There is an uncovered medical need for the treatment of epilepsies. Medication with antiepileptic drugs of either the "old and new generations" although exerts a positive effect on seizure control (at least in about 70% of epileptic patients), deteriorates the cognitive functions (Vermeulen and Aldenkamp 1995 *Epilepsy Res.* 22: 65; Gates 2000 *Epilepsy Behav.* 1: 153; Brunbech and Sabers 2002 *Drugs 62:* 593; Schmidt 2002 *Epilepsy Res.* 50: 21), that may aggravate the cognition decline caused by the illness (Prevey et al. 1998 *Epilepsy Res.* 30: 1; Jokeit and Ebner 1999 *J. Neurol. Neurosurg. Psychiatry* 67: 44; Theodore et al. 1999 *Neurology* 52: 132; Meador 2001 *Epilepsy Behav.* 2: 307). The antiepileptic drugs also cause alterations in the waves of the ABR (Mervaala et al. 1987 *Electroencephalogr. Clin. Neurophysiol.* 68: 475; Armon et al. 1990 *Neurology* 40: 1896; Hirose et al. 1990 *Electroencephalogr. Clin. Neurophysiol.* 75: 543; Yuksel et al. 1995 *Childs Nerv. Syst.* 11: 474; De la Cruz and Bance 1999 *Arch Otolaryngol Head Neck Surg.* 125: 225; Zgorzalewicz and Galas-Zgorzalewicz 2000 *Clin. Neurophysiol.* 111:2150), that can result in a hearing decline (Nekrassov and Sitges 2003 *Epilepsy Res.* 53: 245); and fail to produce a perceptible impact on the prophylaxis of the illness after the first seizure (Hernandez 1997 *Trends Pharmacol. Sci.* 18: 59; Temkin et al. 2001 *Drugs* 61:1045; Schmidt 2002 *Epilepsy Res.* 50: 21).

The available antiepileptic drugs provoke several adverse secondary effects that in many occasions lead the patients to stop the treatment, because of the important alterations that limit their everyday life.

The present invention refers to the use of vinpocetine and the derivates developed from its formula that maintain the same effects for preparing a drug of choice in the treatment of epilepsies. The present invention describes the beneficial action of vinpocetine for preventing the epileptic cortical activity for the ictal and post-ictal periods and for preventing the most important disturbances caused by the illness and aggravated by the available antiepileptic drugs.

### BRIEF DESCRIPTION OF THE FIGURES

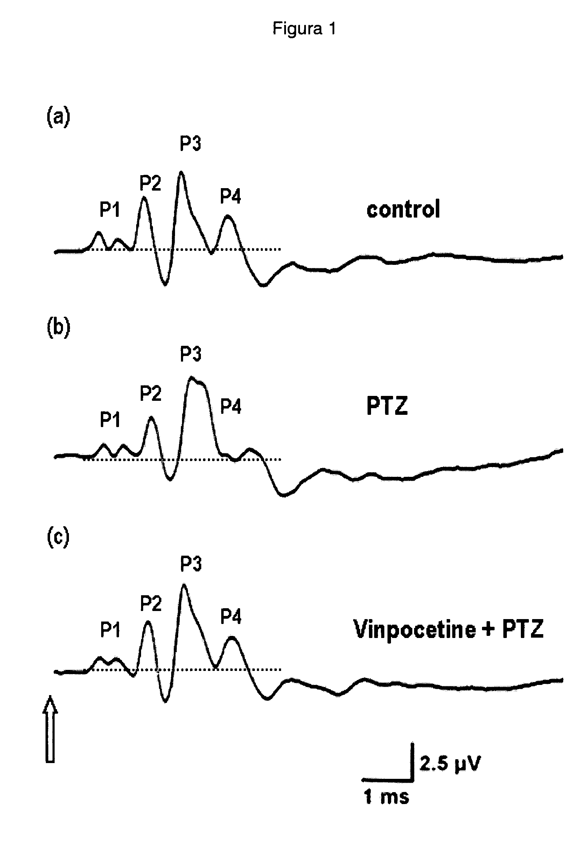

Figure 1. This figure shows the ABR recordings taken in an representative animal before (a) and 50 min after the injection of pentylenetetrazole (PTZ) in an animal pre-injected with vehicle 4 hours before PTZ (b), and in an animal pre-injected with vinpocetine 4 hours before PTZ (c). The animal received a pure tone monaural stimulus of high frequency (8 kHz) and high intensity (100 dB) indicated by the arrow.

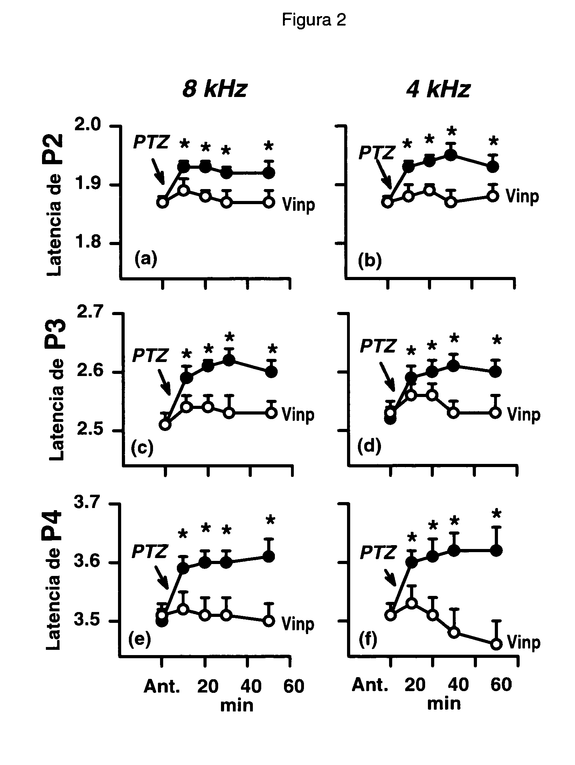

Figure 2. The figure shows that vinpocetine inhibits the increase in the peak latencies of P2, P3 and P4 waves of the ABR induced by PTZ. The latencies of the waves induced by stimuli of 100 dB at tone frequencies of 8 kHz (left graphs) and 4 kHz (right graphs) were determined before (Bef.) and 10, 20, 30 and 50 min after the injection of PTZ in control animals pre-injected with vehicle (black circles) and in animals pre-injected with 2mg/kg vinpocetine (empty circles). Results are the mean ± SEM values of 8 independent animals.

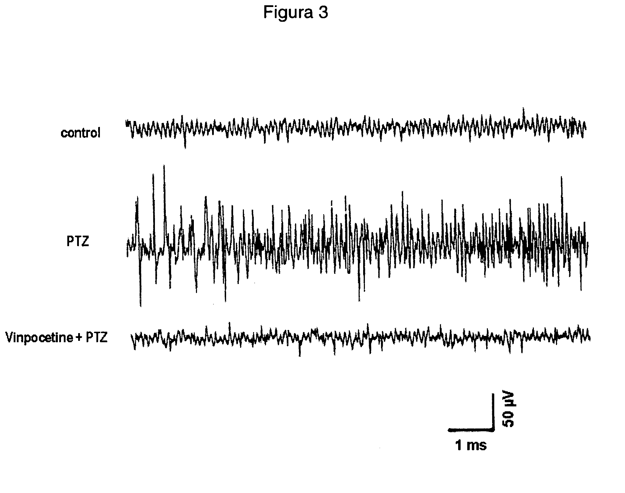

Figure 3. The figure shows that vinpocetine inhibits the changes on the electroencephalogram (EEG) induced by PTZ for the ictal period. The EEG recordings shown were taken in a representative animal before (top trace) and about 2 min after the injection of PTZ in the animal pre-injected with vehicle 4 hours before PTZ (middle trace) or pre-injected with vinpocetine 4 hours before PTZ (bottom trace).

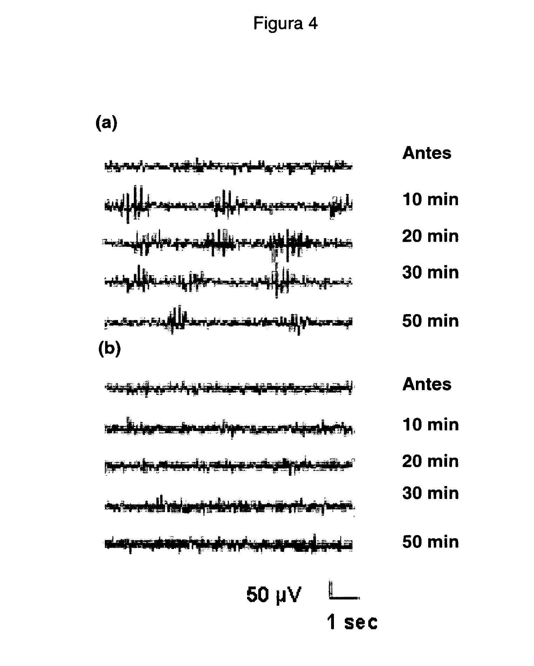

Figure 4. The figure shows that vinpocetine inhibits the changes on the EEG induced by PTZ for the post-ictal period. EEG recordings were taken in a representative animal pre-injected with vehicle before (top trace) and 10, 20, 30 and 50 min after the injection of PTZ. EEG recordings were taken in a representative animal pre-injected with vinpocetine before (top trace) and 10, 20, 30 and 50 min after the injection of PTZ.

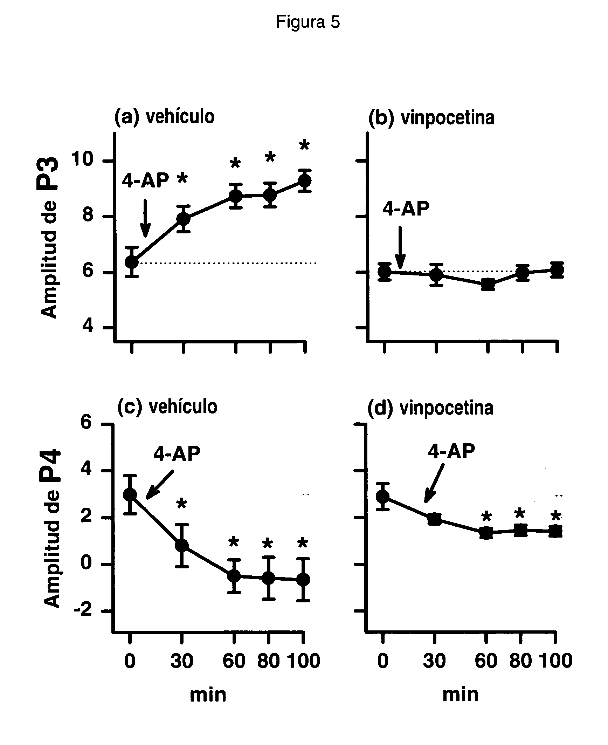

Figure 5. The figure shows that vinpocetine inhibits the alterations in the amplitude of the later ABR waves induced by another convulsive agent, 4-aminopyridine (4-AP). The ABRs were induced by a stimulus of 100 dB at 8 kHz. It is shown that the progressive increase in the amplitude of the P3 wave of the ABR observed after the injection of 4-AP in control animals (a) is eliminated in the animals pre-injected with vinpocetine (b), and that the marked decrease in the amplitude of the P4 wave of the ABR observed after the injection of 4-AP in control animals (c) is considerably reduced in the animals that received vinpocetine (d).

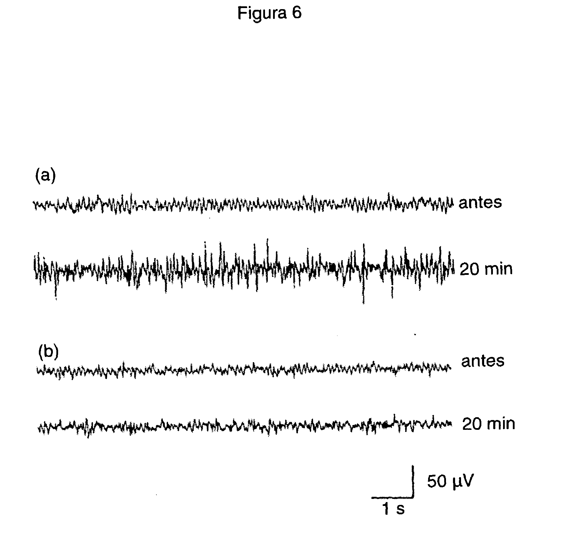

Figure 6. This figure shows that vinpocetine inhibits the changes on the EEG induced by 4-AP for the ictal period. The EEG recordings shown in (a) were taken in a representative animal before and about 20 min after the injection of 4-AP in an animal pre-injected with vehicle. The EEG recordings shown in (b) were taken in a representative animal before and about 20 min after the injection of 4-AP in the animal pre-injected with 2mg/kg vinpocetine.

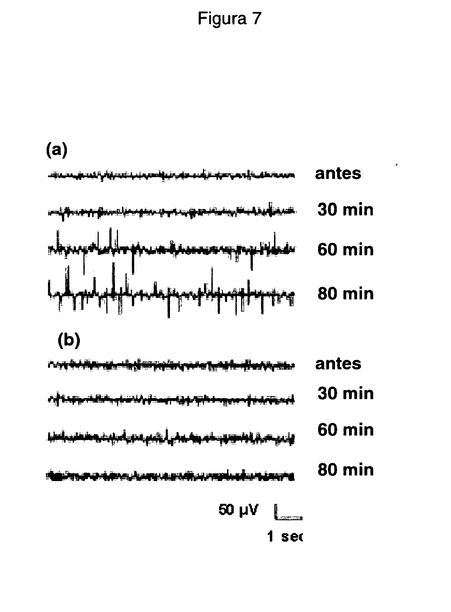

Figure 7. This figure shows that vinpocetine inhibits the changes on the EEG induced by 4-AP for post-ictal period. The EEG recordings shown in (a) were taken in a representative animal pre-injected with vehicle before (top trace) and 30, 60 and 80 min after the injection of 4-AP. The EEG recordings shown in (b) were taken in a representative animal pre-injected with vinpocetine before (top trace) and 30, 60 and 80 min after the injection of 4-AP.

### DETAILED DESCRIPTION OF THE INVENTION

In a previous study we have shown that the alterations in the activity of the lateral and the medial nuclei of the superior olivary complex reflected in abnormalities in the parameters (amplitude and latency) of the later waves of the ABR are connected with the hearing decline caused by epilepsy (Nekrassov and Sitges 2003 *Epilepsy Res.* 53: 245).

The present invention demonstrates that vinpocetine inhibits the alterations in the amplitude and latency of the later ABR waves, as well as the hearing decline and the characteristic epileptic cortical activity observed in two models of experimental animal epilepsy in vivo.

Epilepsy can be induced in experimental animals in vivo either by decreasing the cerebral inhibitory transmission or by increasing the cerebral excitatory transmission. This can be achieved the injection of the GABA antagonist, PTZ or the glutamate releaser, 4-aminopiridine (4-AP), respectively. Our results in the guinea pig show that when vinpocetine is administered at a concentration of 2 mg/kg i.p. 4 hours before the injection of PTZ or 1 hour before the injection of 4-AP, none of these convulsing agents is capable to induce the alterations in amplitude and latency of the later ABR waves, to provoke the hearing decline or to induce the epileptic cortical activity.

In the guinea pig the P3 and P4 waves of the ABR express the activity of the medial and the lateral superior olivary nuclei, respectively (Wada and Starr 1983 *Electroencephalogr. Clin. Neurophysiol.* 56: 326; 56: 340; 56: 352). Among the nuclei of the superior olive, the P4 generator is determinant in sound source localization (Tollin 2003 *Neuroscientist* 9: 127). Changes in these late waves of the ABR indicate retro-cochlear alterations. Vinpocetine cancels all the retro-cochlear abnormalities induced by PTZ or by 4-AP (evidenced by the changes in P3 and/or P4 parameters) and by this mean prevents the hearing decline induced by both convulsing agents.

The available anti epileptic drugs induce alterations in the parameters of the ABR waves and hearing deficits that may aggravate the abnormalities and the hearing loss induced by the illness. Medication with antiepileptic drugs although exerts a positive effect on seizure control also causes secondary adverse effects, among which cognition decline and hearing loss are particularly important. Vinpocetine is well tolerated and without contraindications in humans at doses as high as 60 mg/day (Hindmarch et al. 1991 *Int. Clin. Psychopharmacol.* 6: 31). Our results indicate that vinpocetine at a reasonable dose (2 mg/kg) completely cancels the ictal and post-ictal cortical activity induced by PTZ and 4-AP, as well as the hearing loss induced by the two convulsing agents. The contribution of hearing loss to the decline exerted by epilepsy and by the classic antiepileptic drugs on cognition is prevented by vinpocetine, indicating the advantage that represents the use of vinpocetine as antiepileptic over the classical antiepileptic treatments.

Another problem of the available anti epileptic drugs is that they fail to produce a perceptible impact on epileptogenesis or the progression of the illness. In a previous study we have shown that vinpocetine exerts a long term (more than half a year) protective action over the changes in the ABR waves, the hearing loss, and the mortality induced by the treatment with a high dose of amikacin (Nekrassov and Sitges 2000 *Brain Res.* 868: 222), suggesting that vinpocetine may also exert an important prophylactic action in the treatment of epilepsy.

In summary, our findings indicate that vinpocetine prevents the epileptic cortical activity for the ictal and post-ictal periods, as well as the alterations in the ABR waves, that result in hearing loss and contribute to the adverse effects caused by epilepsy and the available antiepileptic drugs on cognition. These findings, along with the vinpocetine long term protective action indicate that vinpocetine is a better alternative in the treatment and prophylaxis of epilepsies.

### EXAMPLES

Experiments were performed in pigmented adult male guinea pigs initially weighing 349 ± 38 g. ABRs recordings were used to evaluate the hearing status of each animal and EEG recordings to evaluate changes in cortical excitability. The ABR and EEG recordings were obtained following a method that we have previously reported (Nekrassov and Sitges, 2003). The Institutional Animal Use and Care Committee approved all experimental procedures.

Three types of recordings were performed in the anaesthetized animals, namely the ABR recordings elicited by a stimulus of high intensity (100 dB), the ABR recordings for determination of the auditory threshold and the EEG recordings.

Determination of ABR wave parameters. The latency and amplitude of each wave component of the ABR elicited by a stimulus of 100 dB with pure tone frequencies of 4 and 8 kHz was measured in all the ABR recordings obtained under the different experimental conditions at the specified times. The latency of each ABR wave (in ms) refers to the time interval between the onset of the auditory stimulus and the positive peak of the wave. The onset of the stimulus is indicated by the vertical arrow at the bottom of the recordings on Fig. 1. The peak amplitude (in µV) of each wave of the ABR is the difference between the positive peak of the wave and the reference baseline (trace between the stimulus and the appearance of the first ABR wave on Fig. 1).

Determination of ABR thresholds. ABR recordings elicited by stimuli of progressively lower intensity (in dB) were used for determining the hearing threshold. Threshold is defined as the lowest stimulus intensity (in dB) at which the P3 wave of the ABR could still be recorded in three consecutive trials.

Student's t-test was used for the evaluation of the differences between results obtained before and at the specified times after the injection of the convulsing agents. The criterion for statistical significance for all measures was *P* ≤ 0.05. All data are expressed as means ± standard error of the mean. In figures and tables the symbol * is used for indicating statistic significant differences.

### Example 1. Experimental design used for testing the effect of vinpocetine on the changes on the ABR and EEG recordings induced by epilepsy resulting from a reduced cerebral inhibitory transmission.

Eight male guinea pigs were entered into this study. PTZ was dissolved in saline and vinpocetine in a saline acidified (with HCI) adjusted to pH 4 (with NaOH). Four hours after injecting guinea pigs with vehicle (acidified saline used to dissolve vinpocetine) the animals were anaesthetized and a first set of ABR and EEG recordings was taken before the injection (i.p) of the convulsing agent, PTZ. The animals were injected with PTZ (100 mg/kg) and about 2 min after injecting PTZ (ictal period) the EEG recording was taken. Then the other series of ABR and EEG recordings were taken at specific times within the post-ictal period. After two weeks the same series of recordings was taken but instead vehicle the animals were injected with vinpocetine (2 mg/kg) 4 hours before the injection of PTZ.

The next table shows that vinpocetine inhibits the reduction in P4 wave peak amplitude induced by PTZ . In the control animals (pre-injected with vehicle) the amplitude of the P4 wave of the ABR induced by a stimulus of 100 dB at two tone frequencies (8 and 4 kHz) is progressively reduced by PTZ (left columns), whereas in the animals pre-injected with vinpocetine the reduction produced induced by PTZ in P4 amplitude is not observed (right columns). The values shown on the table are the mean ± standard errors of the P4 wave amplitudes in µV obtained from 8 animals before and at the indicated times after the injection of the convulsing agent (PTZ).

| | 8 kHz | 8 kHz |
|---|---|---|
| | PTZ | Vinpocetine & PTZ |
| *Before* | *2.88 ± 0.1* | *2.69 ± 0.1* |
| *10 min* | *2.07 ± 0.4** | *2.56 ± 0.1* |
| *20min* | *1.99 ± 0.3** | *2.71 ± 0.2* |
| *30 min* | *1.92 ± 0.3** | *2.55 ± 0.2* |
| *50 min* | *1.51 ± 0.5** | *2.56 ± 0.1* |

| | 4 kHz | 4 kHz |
|---|---|---|
| | PTZ | Vinpocetine & PTZ |
| *Before* | *3.14 ± 0.1* | *2.82 ± 0.3* |
| *10 min* | *1.70 ± 0.5** | *2.55 ± 0.3* |
| *20min* | *2.12 ± 0.4** | *2.52 ± 0.2* |
| *30min* | *1.84 ± 0.4** | *2.55 ± 0.2* |
| *50min* | *2.11 ± 0.3** | *2.52 ± 0.2* |

Vinpocetine also inhibits the increase produced after the injection of PTZ in the latencies of the ABR waves P2, P3 and P4 induced by a 100 dB stimulus at 4 and 8 kHz tone frequencies (Fig. 2).

The next table shows that vinpocetine inhibits the hearing loss induced by the convulsing agent, PTZ. The marked increase on the auditory threshold at 4 and 8 kHz tone frequencies induced by PTZ (left columns) is not produced in the animals pre-injected with vinpocetine before PTZ administration (right columns). The values shown on the table are the mean ± standard errors of the thresholds in dB obtained in 8 animals.

| | PTZ | Vinpocetine & PTZ |
|---|---|---|
| 8 kHz | | |
| *Before* | *7.0 ± 1.2* | *6.0 ± 1.0* |
| *30 min* | *17 ± 2.0** | *6.0 ± 1.0* |
| *50 min* | *21 ± 2.4** | *6.0 ± 1.0* |

| 4 kHz | | |
|---|---|---|
| *Before* | *21 ± 1.0* | *20 ± 1.6* |
| *30 min* | *28 ± 2.0** | *18 ± 1.2* |
| *50 min* | *29 ± 1.0** | *18 ± 1.2* |

Vinpocetine inhibits all the changes induced by PTZ on the EEG. All the anaesthetized animals injected with PTZ developed generalized seizures. The onset of seizure activity, characterized by repetitive high amplitude spike-sharp wave activity in the EEG tracing appears suddenly within the two first min after PTZ injection. This dramatic change on the cortical activity induced by PTZ in the anaesthetized animals during convulsions is followed by a typical pattern of cortical activity characterized by rhythmic spike bursts of high amplitude. The duration time of this typical pattern of cortical activity, that is not accompanied by convulsions is referred as the post-ictal period.

Vinpocetine completely inhibits the changes on the cortical activity induced by PTZ for the ictal and post-ictal periods. The top traces on Figs. 3 and 4 show the characteristic EEG recordings under control conditions (i.e. before the injection of PTZ). The dramatic changes induced about two minutes after the injection of PTZ on the EEG (ictal period) in the animals pre-injected with vehicle are lost when PTZ is injected in the animals pre-injected with vinpocetine (Fig. 3). In the same way, the changes induced by PTZ 10, 20, 30 and 50 min after its injection (Fig. 4a) are lost in the animals pre-injected with vinpocetine (Fig. 4b).

### Example 2. Experimental design used for testing the effect of vinpocetine on the changes on the ABR and EEG recordings induced by an increased cerebral excitatory transmission.

Five guinea pigs were entered into the study. One hour after injecting guinea pigs with vehicle (acidified saline used to dissolve vinpocetine) the animals were anaesthetized and a first set of ABR and EEG recordings was taken before the injection (i.p) of the convulsing agent, 4-AP. The animals were injected with 4-AP (2 mg/kg) and about 20 min after injecting 4-AP (ictal period) the EEG recording was taken. Then the other series of ABR and EEG recordings were taken at specific times within the post-ictal period. After two weeks the same series of recordings were repeated but instead vehicle the animals were injected with vinpocetine (2 mg/kg) 1 hour before the injection of 4-AP.

Vinpocetine also inhibits the changes in P3 and P4 waves amplitude induced by 4-AP. For instance, the progressive increase in the amplitude of the P3 wave of the ABR produced by 4-AP in control animals (Fig. 5a) is eliminated in the vinpocetine treated animals (Fig. 5b), and the reduction in P4 amplitude produced by 4-AP in control animals (Fig. 5c) is markedly reduced in the vinpocetine treated animals (Fig. 5d).

The following table shows that the increased latency of the P4 wave of the ABR induced by the stimulus of 100 dB at 4 and 8 kHz tone frequencies observed at the indicated times after the injection of 4-AP in control animals (left columns), is lost when 4-AP is injected in the vinpocetine pre-treated animals (right columns).

| | 8 kHz | 8 kHz |
|---|---|---|
| | 4-AP | Vinpocetine & 4-AP |
| *Before* | *3.48 ± 0.06* | *3.48 ± 0.04* |
| *30 min* | *3.51 ± 0.13* | *3.55 ± 0. 07* |
| *60 min* | *3.80 ± 0.10** | *3.45 ± 0.08* |
| *80 min* | *3.80 ± 0.10** | *3.36 ± 0.05* |
| *100 min* | *3.83 ± 0.12** | *3.35 ± 0.07* |

| | 4 kHz | 4 kHz |
|---|---|---|
| | 4-AP | Vinpocetine & 4-AP |
| *Before* | *3.50 ± 0.02* | *3.45 ± 0.06* |
| *30 min* | *3.78 ± 0.09** | *3.37 ± 0.06* |
| *60min* | *3.78 ± 0.09** | *3.54 ± 0.11* |
| *80 min* | *3.78 ± 0.09** | *3.56 ± 0.04* |
| *100 min* | *3.75 ± 0.10** | *3.43 ± 0.09* |

Vinpocetine inhibits the hearing loss induced by 4-AP. The following table shows that the increase on the auditory threshold induced by 4-AP at 8 and 4 kHz tone frequencies in control animals (left columns) is lost in the vinpocetine treated animals (right columns).

| | 4-AP | Vinpocetine & 4-AP |
|---|---|---|
| 8 kHz | | |
| Before | *3.8 ± 1.3* | *2.5 ± 1.4* |
| *30 min* | *10.0 ± 2.0** | *-1.3 ± 2.4* |
| *60 min* | *23.8 ± 6.3** | *0.0 ± 3.5* |

| 4 kHz | | |
|---|---|---|
| Before | *13.8 ± 1.3* | *13.8 ± 1.3* |
| *30min* | *20 ± 2.0** | *11.3 ± 1.3* |
| *60 min* | *30 ± 3.5** | *11.3 ± 1.3* |

Vinpocetine inhibits all the changes induced by 4-AP on the EEG. All anaesthetized animals injected with 4-AP developed generalized seizures, characterized by a repetitive high amplitude spike-sharp wave activity in the EEG tracing that appears about 20 min after the injection of 4-AP. This change on the cortical activity elicited by 4-AP for the ictal period is followed by the post-ictal period characterized by isolated spikes of higher amplitude that appear on the EEG about one hour after the injection of 4-AP. Vinpocetine completely prevents the changes on the cortical activity induced by 4-AP for the ictal and post-ictal periods. The top traces of Fig. 6 show the characteristic EEG recordings taken before the injection of 4-AP in the animals pre-injected with vehicle (a) and in the animals pre-injected with vinpocetine (b). The changes induced by 4-AP on for the ictal period are shown in the second trace on Fig. 6a. In the vinpocetine treated animals 4-AP was unable to induce the ictal activity (second trace on Fig. 6b). Fig 7a shows the post-ictal activity induced by 4-AP, which is lost when the convulsing agent (4-AP) in injected in the animals pre-treated with vinpocetine (Fig. 7b).

## Claims

1. Use of vinpocetine and its derivatives for the fabrication of a medicine useful in the treatment and prophylaxis of neurological diseases, particularly epilepsy and capable of antagonize the alterations in the auditory brainstem response (ABR) waves and the hearing loss that accompany the illness.

2. Use of vinpocetine and its derivatives in accordance with claim 1, as antiepileptic medication **characterized by** the inhibition of the epileptic cortical activity for the ictal and post-ictal periods.

3. Use in accordance with claim 1 of vinpocetine and its derivatives for the treatment of alterations of retrochoclear origin, **characterized by** the inhibition of the alterations of the amplitudes and latencies of the different waves of the ABR.

4. Use in accordance with claim 1 of vinpocetine and its derivatives for the treatment of hearing loss, **characterized by** the inhibition of the increase in the auditory threshold induced by diverse chemical agents, namely pentylenetetrazole, 4-aminopyridine and several aminoglycoside antibiotics.

5. Use in accordance with claim 1 of vinpocetine and its derivatives in the prophylaxis of neurological diseases **characterized by** the long term protective action on the ABR wave alterations induced by several aminoglycoside antibiotics.

6. Use of the medicine of claim 1-5, **characterized** because it can be administered orally or parenterally in a pharmaceutical acceptable vehicle.
